(19) **Europäisches Patentamt / European Patent Office / Office européen des brevets**

(11) **EP 4 279 504 A1**

(12) **EUROPEAN PATENT APPLICATION**
published in accordance with Art. 153(4) EPC

(43) Date of publication:
**22.11.2023 Bulletin 2023/47**

(21) Application number: **22739524.1**

(22) Date of filing: **17.01.2022**

(51) International Patent Classification (IPC):
*C07K 16/18* [(2006.01)]   *G01N 33/531* [(2006.01)]
*G01N 33/574* [(2006.01)]   *C07K 14/47* [(2006.01)]

(52) Cooperative Patent Classification (CPC):
**C07K 14/47; C07K 16/18; G01N 33/531; G01N 33/574**

(86) International application number:
**PCT/JP2022/001385**

(87) International publication number:
**WO 2022/154119 (21.07.2022 Gazette 2022/29)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.01.2021 JP 2021005934**

(71) Applicant: Fujirebio Inc.
**Shinjuku-ku
Tokyo 163-0410 (JP)**

(72) Inventors:
• **NISHII, Tomonori
Tokyo 163-0410 (JP)**
• **SATO, Eriko
Tokyo 163-0410 (JP)**
• **IIDA, Kumiko
Hachioji-shi, Tokyo 192-0031 (JP)**
• **YAGI, Shintaro
Hachioji-shi, Tokyo 192-0031 (JP)**
• **AOYAGI, Katsumi
Tokyo 163-0410 (JP)**

(74) Representative: Hoffmann Eitle
**Patent- und Rechtsanwälte PartmbB
Arabellastraße 30
81925 München (DE)**

Remarks:
The complete document including Reference Table(s) and the Sequence Listing(s) can be downloaded from the EPO website

(54) **METHOD FOR PROCESSING SOLUBLE GPC3-CONTAINING SPECIMENS IN SOLUBLE GPC3 IMMUNOASSAYS**

(57) The present invention provides a method and a reagent useful for testing soluble GPC3. More specifically, the present invention provides the following inventions (1), (2), and (3).
(1) A method for treating a specimen in soluble GPC3 immunoassay, the method including mixing a soluble GPC3-containing specimen with a reducing agent.
(2) A soluble GPC3 immunoassay method including the following (a) and (b):
(a) mixing a soluble GPC3-containing specimen with a reducing agent; and
(b) measuring the amount of soluble GPC3 in the mixture obtained in (a) using one or more kinds of antibodies against soluble GPC3.

(3) A soluble GPC3 immunoassay reagent including the following (a) and (b):
(a) a reducing agent; and
(b) one or more kinds of antibodies against soluble GPC3.

EP 4 279 504 A1

**Description**

FIELD

The present invention relates to a method for treating a soluble glypican-3 (GPC3)-containing specimen in soluble GPC3 immunoassay, and the like.

Background

GPC3 is a protein bound to a cell membrane via a glycosylphosphatidylinositol (GPI) anchor present at a C-terminal of GPC3, belonging to a glypican family which is a proteoglycan having a heparan sulfate chain, and having a molecular weight of about 65 kDa. GPC3 is cleaved between an arginine residue at position 358 and a serine residue at position 359 by an enzyme called Furin in a Golgi body to generate an N-terminal fragment (about 40 kDa) and a GPI anchor-containing C-terminal fragment (about 30 kDa). However, it has been confirmed that such two fragments are usually linked to each other by a disulfide bond. Thus, GPC3 is considered to be bound to a cell membrane via a GPI anchor in a form of a full-length protein including two fragments linked to each other by a disulfide bond.
Since GPC3 is also a protein that is recognized to be specifically expressed in cancer such as liver cancer, development of a method useful for a test of a cancer patient targeting GPC3 is being sought. For example, Patent Literature 1 proposes a method for testing a cancer patient by measuring soluble GPC3. In addition, Patent Literature 2 proposes a method for measuring GPC3 using two different antibodies that bind to different epitopes present in an N-terminal region of GPC3.

CITATION LIST

PATENT LITERATURE

Patent Literature 1: WO 2004/038420 A
Patent Literature 2: WO 2015/097928 A

SUMMARY

TECHNICAL PROBLEM

An object of the present invention is to provide a method and a reagent useful for testing soluble GPC3.

SOLUTION TO PROBLEM

As a result of intensive studies, the present inventors have found that, in soluble GPC3 immunoassay, by treating a specimen with a reducing agent, discrimination accuracy between a positive specimen and a negative specimen can be improved. The present inventors have also found that, in soluble GPC3 immunoassay, by treating a specimen with both a reducing agent and a surfactant, discrimination accuracy between a positive specimen and a negative specimen can be improved, and a matrix effect can be reduced, thereby completing the present invention.
That is, the present invention is as follows.

[1] A method for treating a soluble GPC3-containing specimen in soluble GPC3 immunoassay, the method comprising mixing the soluble GPC3-containing specimen with a reducing agent.
[2] The method according to [1], wherein the reducing agent is used at a final concentration of 0.05 to 1,000 mM.
[3] The method according to [1] or [2], comprising further mixing the soluble GPC3-containing specimen with a surfactant.
[4] The method according to [3], wherein the surfactant is used at a final concentration of 0.005 to 10% by weight.
[5] The method according to any of [1] to [4], wherein the immunoassay is sandwich immunoassay using two or more different kinds of antibodies against two or more different kinds of epitopes in soluble GPC3.
[6] The method according to any of [1] to [5], wherein the soluble GPC3-containing specimen is a blood specimen.
[7] The method according to any of [1] to [6], wherein the soluble GPC3-containing specimen is obtained from a subject suffering from cancer.
[8] The method according to [7], wherein the cancer is liver cancer.

[9] A soluble GPC3 immunoassay method comprising the following (a) and (b):

(a) mixing a soluble GPC3-containing specimen with a reducing agent; and
(b) measuring the amount of soluble GPC3 in the mixture obtained in (a) using one or more kinds of antibodies against soluble GPC3.

[10] The method according to [9], comprising further mixing the soluble GPC3-containing specimen with a surfactant.
[11] The method according to [9] or [10], wherein the measurement is performed by sandwich immunoassay using two or more different kinds of antibodies against two or more different kinds of epitopes in soluble GPC3.
[12] A soluble GPC3 immunoassay reagent comprising the following (a) and (b):

(a) a reducing agent; and
(b) one or more kinds of antibodies against soluble GPC3.

[13] The immunoassay reagent according to [12], further comprising (c) a surfactant.
[14] The immunoassay reagent according to [12] or [13], wherein the one or more kinds of antibodies against soluble GPC3 are two or more different kinds of antibodies against two or more different kinds of epitopes in soluble GPC3, and the immunoassay reagent is a reagent for sandwich immunoassay.
[15] The immunoassay reagent according to any of [12] to [14], wherein the reagent is a reagent for diagnosing cancer.

EFFECTS OF INVENTION

According to the present invention, in soluble GPC3 immunoassay, discrimination accuracy between a positive specimen and a negative specimen can be improved. Therefore, the present invention is useful for achieving high diagnostic sensitivity for a specific state (for example, a disease such as cancer) in soluble GPC3 immunoassay.

BRIEF DESCRIPTION OF DRAWINGS

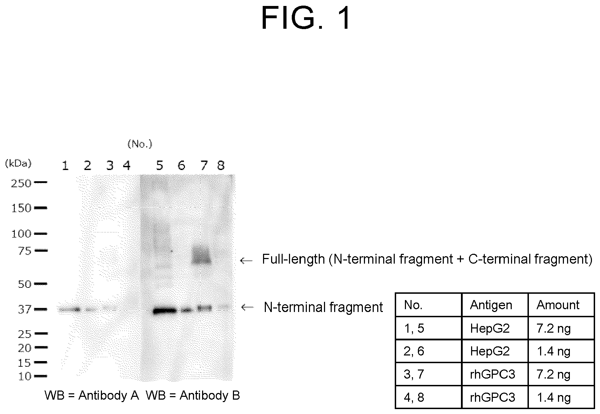

FIG. 1 is a diagram illustrating analysis of recognition sites of an antibody A and an antibody B in an antigenic protein by western blotting (WB). As the antigenic protein, recombinant human GPC3 (rhGPC3) composed of the first to 559th amino acid residues of human GPC3 and a culture supernatant of human hepatoma cell HepG2 highly expressing GPC3 were used. The amount of protein applied in a lane was 1.4 ng/lane or 7.2 ng/lane.

DESCRIPTION OF EMBODIMENTS

The present invention provides a method for treating a specimen in soluble GPC3 immunoassay.
In the present invention, the soluble GPC3 refers to soluble GPC3 secreted from GPC3-expressing cells. As the soluble GPC3, soluble GPC3 derived from any subject can be used. Examples of such a subject include a mammal (for example, a primate such as a human or a monkey; a rodent such as a mouse, a rat, or a rabbit; an ungulate such as a cow, a pig, a goat, a horse, or a sheep, and a carnivore such as a dog or a cat) and a bird (for example, a chicken). The subject is preferably a mammal such as a human. GPC3 is widely conserved in an animal, and an amino acid sequence of GPC3 is highly conserved particularly between mammals. The subject is preferably a human from a viewpoint of clinical application. Therefore, the soluble GPC3 is preferably human soluble GPC3.
The human soluble GPC3 is preferably an N-terminal fragment generated by cleavage between an arginine residue at position 358 and a serine residue at position 359 in a human GPC3 protein (Accession No.: P51654.1) composed of 580 amino acid residues or soluble full-length GPC3 released by cleavage of a GPI anchor present at a C-terminal of the human GPC3 protein. Examples of the soluble full-length GPC3 include a GPC3 fragment in which an N-terminal fragment and a C-terminal fragment generated by cleavage between an arginine residue at position 358 and a serine residue at position 359 in a human GPC3 protein are linked to each other via a disulfide bond. More specifically, such a human soluble GPC3 is (a) an N-terminal fragment composed of amino acid residues at positions 1 to 358 in the amino acid sequence of SEQ ID NO: 1 or a variant thereof, (b) soluble full-length GPC3 in which an N-terminal fragment composed of amino acid residues at positions 1 to 358 in the amino acid sequence of SEQ ID NO: 1 or a variant thereof and a soluble C-terminal fragment composed of amino acid residues at positions 359 to 560 in the amino acid sequence of SEQ ID NO: 1 or a variant thereof are linked to each other by a disulfide bond, or (c) a mutant thereof that can be generated naturally among racial groups and/or individuals. Such a mutant is one having introduction of a mutation (for example, substitution, insertion, or deletion) of one or more amino acid residues which can occur naturally between racial groups and/or individuals into (a) an N-terminal fragment or a variant thereof or (b) soluble full-length GPC3 or a variant thereof. The number of mutations of amino acid residues in such a mutant may be, for example, 1 to 30, preferably 1 to 20, more preferably 1 to 15, still more preferably 1 to 10, and particularly preferably 1, 2, 3, 4, or 5.

In the present invention, the immunoassay refers to soluble GPC3 immunoassay using one or more kinds of antibodies against soluble GPC3. Examples of the antibody include a polyclonal antibody and a monoclonal antibody. The antibody is preferably a monoclonal antibody. The antibody can also be identified by an isotype. Examples of such an isotype include IgG, IgM, IgA, IgD, IgE, and IgY. The antibody is preferably IgG, IgM, or IgA, more preferably IgG or IgM, and still more preferably IgG. Furthermore, the antibody may be a chimeric antibody, a humanized antibody, or a human antibody. The antibody may also be a full-length antibody including a heavy chain and a light chain each including a variable region and a constant region, or a fragment thereof. Examples of the antibody fragment include $F(ab')_2$, Fab', Fab, and Fv. The antibody may also be a single chain antibody (scFv) or a VHH antibody.

The antibody against soluble GPC3 used in the present invention is not particularly limited as long as one or more kinds of antibodies are used, and one kind, two kinds, three kinds, four kinds, or five kinds of antibodies may be used. When two or more kinds of antibodies against soluble GPC3 are used in the present invention, such two or more kinds of antibodies can recognize the same or different epitopes. Such two or more kinds of antibodies may preferably recognize different epitopes. As an epitope available in soluble GPC3 immunoassay (for example, two or more different kinds of epitopes available in sandwich assay) and an antibody against the epitope, various epitopes and antibodies are known (see, for example, WO 2015/097928 A, WO 2004/038420 A, or WO 2004/022739 A). Therefore, such known epitopes and antibodies against the known epitopes may be used in the present invention. In addition, since an antibody against soluble GPC3 is commercially available, a commercially available antibody can also be used in the present invention. Use of one kind or two kinds of antibodies against soluble GPC3 is preferred from a viewpoint of, for example, simple immunoassay. The antibody against soluble GPC3 used in the present invention is preferably an antibody having an ability to bind to a region in an N-terminal fragment of GPC3, and more preferably an antibody having an ability to specifically bind to a region in the N-terminal fragment of GPC3.

The immunoassay can be performed by any immunoassay using one or more kinds of antibodies against soluble GPC3. Examples of such immunoassay include chemiluminescence immunoassay (CLIA) [for example, chemiluminescent enzyme immunoassay (CLEIA)], immunoturbidimetry (TIA), enzyme immunoassay (EIA) (for example, direct ELISA, indirect ELISA, competitive ELISA, or sandwich ELISA), radioimmunoassay (RIA), a latex agglutination reaction method, fluorescence immunoassay (FIA), an immunochromatography method, western blotting, and immunostaining.

The immunoassay can also be performed by any immunoassay using two or more kinds of antibodies against soluble GPC3, including a labeled antibody and an immobilized antibody against soluble GPC3. The labeled antibody is an antibody labeled with a labeling substance or an antibody to be labeled with a labeling substance in an immunoassay step. Examples of the labeling substance include a fluorescent substance, a luminescent substance, a dye, and an enzyme. The immobilized antibody is an antibody immobilized on a solid phase or an antibody to be immobilized on a solid phase in an immunoassay step. Examples of the solid phase include particles (for example, microparticles, nanoparticles, microbeads, nano-beads, microspheres, or nanospheres), a support (for example, a membrane), and a substrate (for example, a plate). The solid phase may be a solid phase having magnetism (for example, magnetic particles).

The immunoassay can also be performed in any manner. Examples of such a manner include a direct method, an indirect method, a competitive method, and a sandwich method. The immunoassay may be preferably performed by sandwich immunoassay using two or more different kinds of antibodies against two or more different kinds of epitopes of soluble GPC3. In such sandwich immunoassay, two or more kinds of antibodies including a labeled antibody and an immobilized antibody against soluble GPC3 are used as the two or more different kinds of antibodies against two or more different kinds of epitopes of soluble GPC3. The immunoassay may be performed by sandwich immunoassay using two kinds of antibodies (labeled antibody and immobilized antibody) against two different kinds of epitopes in soluble GPC3 from a viewpoint of, for example, simple immunoassay. The two kinds of antibodies (labeled antibody and immobilized antibody) against two different kinds of epitopes of soluble GPC3 may be two kinds of antibodies against different epitopes of an N-terminal fragment or two kinds of antibodies against different epitopes of a C-terminal fragment. Alternatively, such two kinds of antibodies may be a combination of one kind of antibody against an epitope of an N-terminal fragment and one kind of antibody against an epitope of a C-terminal fragment. Such two kinds of antibodies are preferably two kinds of antibodies against different epitopes of an N-terminal fragment.

A method for treating a soluble GPC3-containing specimen by the present invention includes mixing the soluble GPC3-containing specimen with a reducing agent. As a result, a mixture of the soluble GPC3-containing specimen and the reducing agent is generated.

The soluble GPC3-containing specimen is any specimen containing the soluble GPC3 described above. Examples of the soluble GPC3-containing specimen include a liquid specimen obtained from a subject (for example, blood, lymph, urine, milk, saliva, or a tear fluid), an extracted liquid specimen of a tissue obtained from a subject, a washing liquid specimen that can be collected from a subject (for example, those obtained by washing a mucosal tissue of a bronchus or the like), a specimen obtained from a cell culture derived from a subject, a specimen containing recombinant soluble GPC3 (for example, a soluble GPC3 preparation), and liquid specimens obtained by treating (for example, fractionating) these specimens. The soluble GPC3-containing specimen is preferably a blood specimen (for example, whole blood, serum, or plasma) from a viewpoint of, for example, simple availability of a specimen abundantly containing soluble GPC3.

In a specific embodiment, the soluble GPC3-containing specimen may be a specimen obtained from a subject in a specific state. Examples of such a subject include a subject suffering from a specific disease and a subject who may suffer from a specific disease. Examples of the specific disease include cancer (for example, liver cancer, prostate cancer, or malignant melanoma) and a liver disease (for example, hepatitis or liver cirrhosis) (for example, see WO 2004/038420 A, WO 2007/081790 A, WO 2005/039380 A, or Detection of glypican-3-specific CTLs in chronic hepatitis and liver cirrhosis, Oncology Reports 22, p. 149-54, 2009).

When the soluble GPC3-containing specimen is mixed with a reducing agent, any reducing agent can be used. Examples of such a reducing agent include 2-(dimethylamino) ethanethiol (DEAET), tris(2-carboxyethyl) phosphine (TCEP), 2-mercaptoethylamine, 2-mercaptoethanol, dithiothreitol, thioglycerol, sodium sulfite, a borohydride, and salts thereof. Examples of the salts include a metal salt (for example, a monovalent metal salt such as a sodium salt or a potassium salt, or a divalent metal salt such as a calcium salt or a magnesium salt), an inorganic salt (for example, a halide salt such as a fluoride, a chloride, a bromide, or an iodide, or an ammonium salt), an organic salt (for example, an ammonium salt having an alkyl group as a substituent), and an acid addition salt (for example, a salt with an inorganic acid such as sulfuric acid, hydrochloric acid, hydrobromic acid, nitric acid, or phosphoric acid, or a salt with an organic acid such as acetic acid, oxalic acid, lactic acid, citric acid, trifluoromethanesulfonic acid, or trifluoroacetic acid). DEAET, TCEP, or salts thereof are preferable from a viewpoint of, for example, use of a reducing agent that is highly stable in a solution.

The reducing agent can be used at a final concentration at which a detection signal intensity (for example, count) from a negative specimen can be reduced. The final concentration is a concentration at the time of mixing. Therefore, the final concentration can also be expressed as a concentration in a mixture generated by mixing. Such a final concentration is, for example, 0.05 to 1,000 mM, preferably 0.5 to 500 mM, more preferably 1 to 300 mM, and still more preferably 3 to 200 mM.

Mixing of the soluble GPC3-containing specimen and the reducing agent may include further mixing the soluble GPC3-containing specimen with a surfactant. In such a case, a mixture of the soluble GPC3-containing specimen, the reducing agent, and the surfactant is generated.

Examples of the surfactant include a nonionic surfactant, a zwitterionic surfactant, an anionic surfactant, a cationic surfactant, and salts thereof. The salts are similar to those described above. Examples of the nonionic surfactant include a polyoxyethylene sorbitan fatty acid ester, a polyoxyethylene alkylphenyl ether, and a polyoxyethylene alkyl ether. Examples of the polyoxyethylene sorbitan fatty acid ester include polyoxyethylene sorbitan monolaurate (Tween 20), polyoxyethylene sorbitan monopalmitate (Tween 40), polyoxyethylene sorbitan monostearate (Tween 60), and polyoxyethylene sorbitan monooleate (Tween 80). Examples of the polyoxyethylene alkylphenyl ether include polyoxyethylene (10) octylphenyl ether (Triton X-100), polyoxyethylene (8) octylphenyl ether (Triton X-114), polyoxyethylene (30) octylphenyl ether (Triton X-305), and polyoxyethylene (40) octylphenyl ether (Triton X-405). Examples of the polyoxyethylene alkyl ether include polyoxyethylene (23) lauryl ether (Brij 35) and polyoxyethylene (20) cetyl ether (Brij 58). Preferred examples of the nonionic surfactant include polyoxyethylene sorbitan monolaurate (Tween 20) and polyoxyethylene (10) octylphenyl ether (Triton X-100). Examples of the zwitterionic surfactant include a sulfobetaine type surfactant. Examples of the sulfobetaine type surfactant include 3-[(3-cholamidopropyl) dimethylammonio]-1-propanesulfonate (CHAPS), 3-[3-cholamidopropyl] dimethylammonio)-2-hydroxypropanesulfonate (CHAPSO), N-dodecyl-N,N-dimethyl-3-ammonio-1-propanesulfonate, N-tetradecyl-N,N-dimethyl-3-ammonio-1-propanesulfonate, and N-hexadecyl-N,N-dimethyl-3-ammonio-1-propanesulfonate. Examples of the anionic surfactant include sodium dodecyl sulfate (SDS), sodium N-lauroyl sarcosine (NLS) lithium dodecyl sulfate, sodium dodecylbenzene sulfonate, and a deoxycholate. Examples of the cationic surfactant include decyltrimethylammonium chloride, dodecyltrimethylammonium chloride, tetradecyltrimethylammonium chloride, hexadecyltrimethylammonium chloride, decyltrimethylammonium bromide, dodecyltrimethylammonium bromide, tetradecyltrimethylammonium bromide, and hexadecyltrimethylammonium bromide. The surfactant is preferably a nonionic surfactant, a zwitterionic surfactant, or a salt thereof.

The surfactant can be used at a final concentration at which an effect of the reducing agent to reduce a detection signal intensity from a negative specimen can be enhanced and/or a matrix effect of the specimen can be reduced. The final concentration is a concentration at the time of mixing. Therefore, the final concentration can also be expressed as a concentration in a mixture generated by mixing. Such a final concentration is, for example, 0.005 to 10% by weight, preferably 0.01 to 9% by weight, more preferably 0.02 to 7.5% by weight, still more preferably 0.05 to 6% by weight, and particularly preferably 0.05 to 5% by weight.

The reducing agent and the surfactant can be preferably used at a ratio at which an effect can be enhanced in reducing a detection signal intensity from a negative specimen and/or reducing a matrix effect of the specimen. Such a ratio can be defined by a concentration range of the surfactant per 1 mM of the reducing agent. The concentration range of the surfactant per 1 mM of the reducing agent is, for example, 0.000025 to 3% by weight, preferably 0.00005 to 0.1% by weight, more preferably 0.0005 to 0.01% by weight, and still more preferably 0.005 to 0.05% by weight.

When the soluble GPC3-containing specimen is mixed with both the reducing agent and the surfactant, mixing can be performed simultaneously or separately. When mixing is performed simultaneously, the soluble GPC3-containing specimen can be mixed with a mixture of the reducing agent and the surfactant. When mixing is performed separately, the soluble GPC3-containing specimen may be mixed with the reducing agent first and then with the surfactant, or may be mixed with the surfactant first and then with the reducing agent.

The reducing agent and/or the surfactant can be used by being dissolved in an aqueous solution. Examples of such an aqueous solution include water (for example, distilled water, sterile water, sterile distilled water, or pure water) and a buffer. Examples of the buffer include a phosphate buffer, a MES buffer, a citrate buffer, a Tris buffer, a carbonate buffer, a HPEPS buffer, and a MOPS buffer. The pH of the buffer varies depending on a factor such as the type or concentration of the reducing agent, but may be 1.0 to 9.0 (preferably 4.0 to 6.0) from a viewpoint of, for example, improving an effect of the reducing agent. The aqueous solution is preferably a buffer from a viewpoint of, for example, stably exhibiting an effect of the reducing agent in a desired pH range. The aqueous solution may contain another component such as an organic solvent (for example, an alcohol). A ratio in a volume to be mixed between the soluble GPC3-containing specimen and the aqueous solution containing the reducing agent and/or the surfactant (soluble GPC3-containing specimen : aqueous solution containing reducing agent and/or surfactant) is, for example, 10 : 1 to 1 : 10, preferably 5 : 1 to 1 : 5, and more preferably 2 : 1 to 1 : 2.

Mixing is performed under a condition sufficient for a treatment of the specimen with the reducing agent alone or with both the reducing agent and the surfactant. Such a temperature condition is, for example, 15 to 60°C, preferably 20 to 50°C, and more preferably 25 to 45°C. Mixing time is, for example, 30 seconds or less. The mixing time is preferably 20 seconds or less, and more preferably 15 seconds or less from a viewpoint of, for example, rapid treatment. By performing mixing under such a condition, a detection signal intensity from a negative specimen can be reduced.

The method of the present invention may include further incubating the mixture after mixing. Incubation time varies depending on a factor such as the type and concentration of a reducing agent, presence or absence of combined use of a surfactant, the type and concentration of a surfactant, mixing time, the degree of reduction desired in a detection signal intensity, or time required for measurement (immunoassay) using an antibody when the measurement is performed, but is, for example, 120 minutes or less, preferably 60 minutes or less, and more preferably 30 minutes or less. The incubation time is still more preferably 20 minutes or less and particularly preferably 10 minutes or less, or may be 5 minutes or less from a viewpoint of, for example, rapid treatment. Incubation temperature is similar to the temperature condition in the mixing described above.

The present invention also provides a soluble GPC3 immunoassay method including the following (a) and (b) :

(a) mixing a soluble GPC3-containing specimen with a reducing agent; and
(b) measuring the amount of soluble GPC3 in the mixture obtained in (a) using one or more kinds of antibodies against soluble GPC3.

The definitions, examples, and preferred examples of various elements in the soluble GPC3 immunoassay method are similar to those described in the method for treating a soluble GPC3-containing specimen according to the present invention.

Step (a) can be performed in a similar to the method for treating a soluble GPC3-containing specimen according to the present invention. Step (b) can be performed by the above-described immunoassay. Steps (a) and (b) can be performed in parallel or separately. For example, when steps (a) and (b) are performed in parallel, by simultaneously mixing a soluble GPC3-containing specimen, a reducing agent (and a surfactant), and one or more kinds of antibodies against soluble GPC3, a treatment of the soluble GPC3-containing specimen with the reducing agent (and the surfactant) and an antigen-antibody reaction can be simultaneously performed. However, in order to sufficiently reduce a detection signal intensity (for example, count) from a negative specimen, it is preferable to sufficiently treat a soluble GPC3-containing specimen with a reducing agent (and a surfactant), and then to measure the amount of soluble GPC3 by an antigen-antibody reaction using one or more kinds of antibodies against soluble GPC3. In addition, when the antibody used in the present invention includes a disulfide bond, if the antibody and a reducing agent are allowed to coexist for a long time, the reducing agent breaks the antibody by cleavage of the disulfide bond in the antibody, and therefore measurement accuracy of the immunoassay may be affected. Therefore, when the antibody used in the present invention includes a disulfide bond, steps (a) and (b) are preferably performed separately. Of course, when the antibody used in the present invention does not include a disulfide bond (for example, a single chain antibody), it is also preferable to perform steps (a) and (b) in parallel.

The present invention further provides a soluble GPC3 immunoassay reagent including the following (a) and (b) :

(a) a reducing agent; and
(b) one or more kinds of antibodies against soluble GPC3.

The reagent of the present invention may further include (c) a surfactant.

Definitions, examples, and preferred examples of the reducing agent, antibody, and surfactant as components (a) to (c) are similar to those described in the method for treating a soluble GPC3-containing specimen according to the present invention.

In a specific embodiment, the reagent of the present invention may be a reagent in which one or more kinds of antibodies against soluble GPC3 are two or more different kinds of antibodies against two or more different kinds of epitopes in soluble GPC3 and which is a reagent for sandwich immunoassay. Such a reagent preferably includes a labeled antibody and/or an immobilized antibody against soluble GPC3 as the two or more kinds of antibodies. Alternatively, when such a reagent does not include a labeled antibody labeled with a labeling substance and/or an immobilized antibody immobilized on a solid phase, the reagent may include a labeling substance and/or a solid phase. Examples of the labeling substance include a fluorescent substance, a luminescent substance, a dye, and an enzyme. When the labeling substance is an enzyme, such a reagent may include a substrate of the enzyme (for example, a substrate that generates a detection signal, a substrate that is converted by the enzyme into a product that generates a detection signal, or a substrate in a reaction that can be conjugated with another enzymatic reaction that uses a substrate that generates a detection signal or a substrate that is converted by the enzyme into a product that generates a detection signal). The solid phase is similar to that described above.

The reagent of the present invention can be used for determination of a specific state (for example, diagnosis of a disease). The specific state (for example, a disease) is similar to that described above. The reagent of the present invention can be preferably used for diagnosis of cancer such as liver cancer or prostate cancer.

EXAMPLES

Hereinafter, the present invention will be described in more detail with reference to Examples, but the present invention is not limited to these Examples.

Reference Example 1: Confirmation of reactivity between glypican-3 (GPC3) and antibody

Recognition sites of anti-GPC3 antibody A and anti-GPC3 antibody B (both of which are monoclonal IgG antibodies) were analyzed by western blotting using a recombinant human GPC3 (rhGPC3) (R & D Systems) antigen composed of the first to 559th amino acid residues of GPC3 or a culture supernatant of human hepatoma cell HepG2 highly expressing GPC3.

A sample buffer for SDS-PAGE was added to each of the rhGPC3 and the HepG2 culture supernatant, and each of rhGPC3 and the HepG2 culture supernatant was applied to 5 to 20% polyacrylamide gel (superset ace 5 to 20%, WAKO) such that 1.4 ng protein/lane and 7.2 ng protein/lane were obtained. After electrophoresis (30 mA, 60 minutes), a protein was transferred to a blotting membrane (Immobilon ISEQ, Merck Millipore) using a transblot (registered trademark) SD semi-dry electrophoretic transfer cell (Bio-Rad) (15 V, 60 minutes). The blotting membrane was lightly washed with TBS-T and then shaken in a blocking liquid (0.5% ECL Block (GE Healthcare Life Sciences), 0.5% BSA, TBS-T) for one hour at room temperature. The blotting membrane was washed with TBS-T twice and then shaken overnight at 4°C in a solution containing anti-GPC3 antibody A and anti-GPC3 antibody B each diluted to 1 $\mu$g/mL with a reaction liquid (obtained by diluting the blocking liquid two times with TBS-T). The blotting membrane was washed with TBS-T four times, then shaken for one hour in a solution containing POD-labeled anti-mouse F(ab')$_2$ (Jackson) diluted 20,000 times with the reaction liquid, and then washed with TBS-T. A color development reaction was performed using SuperSignal West Femto Maximum Sensitivity Substrate (Thermo Fisher Scientific), and development was performed using a chemiluminescence imaging system (FUSION SYSTEM, Vilber Lourmat). Analysis results of western blotting are presented in FIG. 1.

As a result, both anti-GPC3 antibody A and anti-GPC3 antibody B reacted with a band of 40 kDa corresponding to an N-terminal fragment of GPC3 (a fragment on the N-terminal side from the 358th amino acid residue) (FIG. 1). This indicates that anti-GPC3 antibody A and anti-GPC3 antibody B recognize an N-terminal region of GPC3. Therefore, it has been confirmed that anti-GPC3 antibody A and anti-GPC3 antibody B recognize soluble GPC3.

Reference Example 2: Preparation of anti-GPC3 antibody immobilized on particles

Anti-GPC3 antibody A was added to magnetic particles in a 10 mM MES buffer (pH 5.0) to obtain a suspension containing 0.2 mg/mL anti-GPC3 antibody A and 0.01 g/mL magnetic particles. The suspension was incubated at 5°C for one hour while being gently stirred to make anti-GPC3 antibody A immobilized on the magnetic particles. Thereafter, the magnetic particles were magnetically collected with a magnet, and the magnetic particles were washed with a washing liquid (50 mM Tris buffer, 150 mM NaCl, 2.0% BSA, pH 7.2) to obtain anti-GPC3 antibody A immobilized on particles. In measurement, the anti-GPC3 antibody A immobilized on particles were suspended in a particle diluent (50 mM Tris buffer, 1 mM EDTA2Na, 0.1% NaN$_3$, 2.0% BSA, pH 7.2).

Reference Example 3: Preparation of alkaline phosphatase-labeled anti-GPC3 antibody

Desalted alkaline phosphatase (ALP) and N-(4-maleimidobutyryloxy)-succinimide (GMBS) (final concentration: 0.3 mg/mL) were mixed, and the mixture was allowed to stand at 30°C for one hour to maleimidate the ALP. Next, anti-GPC3 antibody B converted into Fab' and the maleimidated ALP were mixed at a molar ratio of 1 : 1 in a coupling reaction liquid (100 mM phosphate buffer, 1 mM EDTA2Na, pH 6.3), and reacted at 25°C for one hour. A main peak was fractionated and purified with a purification buffer (50 mM MES buffer, 150 mM NaCl, 0.1% NaN$_3$, pH 8.0) at a flow rate of 0.5 mL/min using column chromatography of Superdex200 10/300 (GE Healthcare) to obtain ALP-labeled anti-GPC3 antibody B. In measurement, ALP-labeled anti-GPC3 antibody B was suspended in a labeled body diluent (50 mM MES buffer, 150 mM NaCl, 0.3 mM ZnCl$_2$, 1 mM MgCl$_2$, 0.1% NaN$_3$, 2.0% BSA, pH 6. 8) .

Reference Example 4: Measurement of soluble GPC3

20 μL of pretreatment liquid was dispensed into a reaction tank, and then 20 μL of a specimen was dispensed into the reaction tank. The mixture of the pretreatment liquid and the specimen was incubated at 37°C for 6.5 minutes, then 50 μL of anti-GPC3 antibody A immobilized on particles was dispensed into the reaction tank, and the mixture was stirred. The mixture was incubated at 37°C for eight minutes and perform B/F separation and washing. 50 μL of ALP-labeled anti-GPC3 antibody B was dispensed into the reaction tank, and then the mixture was stirred. The mixture was incubated at 37°C for eight minutes and perform B/F separation and washing. Thereafter, 200 μL of Lumipulse substrate liquid containing 3-(2'-spiroadamantane)-4-methoxy-4-(3''-phosphoryloxy) phenyl-1,2-dioxetane disodium salt (AMPPD) as a chemiluminescent substrate was dispensed into the reaction tank, and the mixture was stirred. Thereafter, the mixture was incubated at 37°C for four minutes, and then the amount of luminescence was measured with a luminometer. The actual measurement was performed with a fully automated chemiluminescent enzyme immunoassay system (LUMIPULSE L2400 (manufactured by Fujirebio Inc.)).

Example 1: Pretreatment of specimen with reducing agent DEAET in measurement of soluble GPC3

A serum specimen derived from a healthy person (negative specimen) and a serum specimen derived from a liver cancer patient who was positive for α-fetoprotein (AFP) (Trina Bioreactives) (positive specimen) were used as specimens for evaluation of soluble GPC3. As a pretreatment liquid, a phosphate buffer containing 6.25 mM to 600 mM 2-(dimethylamino) ethanethiol hydrochloride (DEAET) (10 mM phosphate buffer, 6.25 to 600 mM DEAET, pH 6.0) was used. As a control liquid, a 10 mM phosphate buffer (pH 6.0) was used. Each of the negative specimen, the positive specimen, and the buffer sample (10 mM phosphate buffer) was mixed with the pretreatment liquid at a volume ratio of 1 : 1, and reacted at 37°C for 6.5 minutes (with pretreatment). Similarly, each of the negative specimen, the positive specimen, and the buffer sample was mixed with the control liquid, and reacted (without pretreatment).

For each of the specimens, soluble GPC3 was measured by the measurement method described in Reference Example 4. The count of each of the specimens is presented in Table 1. In addition, (1) a lowering rate (%), (2) a count difference (%) between the positive and the negative, and (3) a matrix difference (%) calculated from the count by the following calculation formulas are presented in Table 1.

Calculation formulas

(1) Lowering rate (%) = 100 - (count in a case of "with pretreatment"/count in a case of "without pretreatment" at each DEAET concentration) × 100

(2) Count difference (%) between positive and negative = (count average value of positive specimen)/(count average value of negative specimen) × 100

$$(3) \text{ Matrix difference } (\%) = (\text{count of buffer sample})/(\text{count average value of negative specimen}) \times 100$$

The lowering rate (%) is an index for evaluating an influence of the pretreatment on an individual specimen. As the lowering rate of the negative specimen is larger and the lowering rate of the positive specimen is smaller, a difference between the measured value of the negative specimen and the measured value of the positive specimen is larger, and the negative specimen and the positive specimen can be more easily distinguished from each other. Therefore, high diagnostic sensitivity can be achieved. Therefore, a larger lowering rate of the negative specimen and a smaller lowering rate of the positive specimen are more useful as pretreatment conditions of the measurement system of soluble GPC3.

The count difference (%) between the positive and the negative is an index for evaluating the count difference between the positive specimen and the negative specimen by the pretreatment. A larger count difference (%) between the positive and the negative is more useful for the measurement system of soluble GPC3 because higher diagnostic sensitivity can be achieved.

The matrix difference (%) is an index for evaluating a count difference between the buffer sample and the negative specimen. In immunoassay using a specimen, an immune reaction may be affected by a substance contained in the specimen (matrix effect). The closer the matrix difference (%) is to 100%, the more the matrix effect can be reduced. The reduced matrix effect is useful for the measurement system of soluble GPC3 because true value output independent of the type of specimen can be achieved.

[Table 1]

Table 1. Pretreatment of specimens with DEAET

| | | Without Pretreatment | With Pretreatment | | | | | | | | |
|---|---|---|---|---|---|---|---|---|---|---|---|
| | DEAET (mM) | 0 | 6.25 | 12.5 | 25 | 50 | 100 | 200 | 400 | 600 |
| Count | NS-1<br>NS-2 | 11,875<br>13,625 | 8,984<br>9,670 | 8,294<br>8,900 | 8,897<br>9,296 | 8,060<br>8,535 | 6,341<br>6,310 | 5,329<br>4,562 | 3,880<br>3,748 | 4,124<br>3,228 |
| | PS-1<br>PS-2 | 21,323<br>29,252 | 19,915<br>25,887 | 19,864<br>25,875 | 20,831<br>27,279 | 21,656<br>28,061 | 21,721<br>27,922 | 19,440<br>26,749 | 13,585<br>18,975 | 9,773<br>11,827 |
| Lowering rate (%) | NS-1<br>NS-2 | | 24.3<br>29.0 | 30.2<br>34.7 | 25.1<br>31.8 | 32.1<br>37.4 | 46.6<br>53.7 | 55.1<br>66.5 | 67.3<br>72.5 | 65.3<br>76.3 |
| | PS-1<br>PS-2 | | 6.6<br>11.5 | 6.8<br>11.5 | 2.3<br>6.7 | -1.6<br>4.1 | -1.9<br>4.5 | 8.8<br>8.6 | 36.3<br>35.1 | 54.2<br>59.6 |
| Count difference (%) between positive and negative | PS-1,2/NS-1,2 | 198 | 246 | 266 | 264 | 300 | 392 | 467 | 427 | 294 |
| Count | BS<br>Mean of NS-1,2 | 1,734<br>12,750 | 1,848<br>9,327 | 1,748<br>8,597 | 1,740<br>9,097 | 1,727<br>8,298 | 1,707<br>6,326 | 1,895<br>4,946 | 1,982<br>3,814 | 2,209<br>3,676 |
| Matrix difference (%) | BS /Mean of NS-1,2 | 14 | 20 | 20 | 19 | 21 | 27 | 38 | 52 | 60 |
| NS: Negative Specimen<br>PS: Positive Specimen<br>BS: Buffer Sample | | | | | | | | | | | |

As presented in Table 1, when the negative specimen was pretreated with DEAET, the count of the negative specimen decreased at each of the examined concentrations (6.25 mM to 600 mM), and a lowering rate thereof was very large. Meanwhile, when the positive specimen was pretreated with DEAET, the count of the positive specimen did not decrease or a lowering rate thereof was smaller than that of the negative specimen even if the count decreased. In particular, when DEAET was added at a concentration of 50 mM to 400 mM, a count difference (%) between the positive and the negative was very large. This indicates that the pretreatment of the specimen with the reducing agent DEAET can improve discrimination accuracy between the positive specimen and the negative specimen in the measurement of soluble GPC3. In addition, when the negative specimen was pretreated with DEAET, the matrix difference approached 100% and the matrix effect was reduced. Therefore, it has been confirmed that the pretreatment of the specimen with the reducing agent DEAET is useful for the measurement of soluble GPC3.

Example 2: Pretreatment of specimen with reducing agent 2MEA in measurement of soluble GPC3

As a pretreatment liquid, a phosphate buffer containing 6.25 mM to 100 mM 2-mercaptoethylamine hydrochloride (2MEA) (10 mM phosphate buffer, 6.25 to 100 mM 2MEA, pH 6.0) was used. As a control liquid, a 10 mM phosphate buffer (pH 6.0) was used. Each of the negative specimen, the positive specimen, and the buffer sample was mixed with the pretreatment liquid at a volume ratio of 1 : 1, and reacted at 37°C for 6.5 minutes (with pretreatment). Similarly, each of the negative specimen, the positive specimen, and the buffer sample was mixed with the control liquid, and reacted (without pretreatment).

For each of the specimens, soluble GPC3 was measured by the measurement method described in Reference Example 4. The count of each of the specimens is presented in Table 2. In addition, as in Example 1, a lowering rate (%), a count difference (%) between the positive and the negative, and a matrix difference (%) were calculated.

[Table 2]

Table 2. Pretreatment of specimens with 2MEA

| | | Without Pretreatment | With Pretreatment | | | | |
|---|---|---|---|---|---|---|---|
| | 2MEA (mM) | 0 | 6.25 | 12.5 | 25 | 50 | 100 |
| Count | NS-3 NS-4 | 5,747 7,418 | 4,671 5,328 | 3,817 4,431 | 3,421 3,790 | 3,240 3,374 | 3,029 3,226 |
| | PS-1 PS-2 | 18,169 22,033 | 17,119 22,138 | 16,250 21,297 | 16,057 20,697 | 15,046 20,338 | 14,178 18,615 |
| Lowering rate (%) | NS-3 NS-4 | | 18.7 28.2 | 33.6 40.3 | 40.5 48.9 | 43.6 54.5 | 47.3 56.5 |
| | PS-1 PS-2 | | 5.8 -0.5 | 10.6 3.3 | 11.6 6.1 | 17.2 7.7 | 22.0 15.5 |
| Count difference (%) between positive and negative | PS-1,2 / NS-3,4 | 305 | 393 | 455 | 510 | 535 | 524 |
| Count | BS Mean of NS-3,4 | 1,336 6,583 | 1,478 5,000 | 1,511 4,124 | 1,710 3,606 | 1,659 3,307 | 1,680 3,128 |
| Matrix difference (%) | BS / Mean of NS-1,2 | 20 | 30 | 37 | 47 | 50 | 54 |
| NS: Negative Specimen PS: Positive Specimen BS: Buffer Sample | | | | | | | |

As presented in Table 2, when the negative specimen was pretreated with 2MEA, the count of the negative specimen decreased at each of the examined concentrations (6.25 mM to 100 mM), and a lowering rate thereof was very large. Meanwhile, when the positive specimen was pretreated with 2MEA, the count of the positive specimen did not decrease or a lowering rate thereof was smaller than that of the negative specimen even if the count decreased. This indicates that the pretreatment of the specimen with the reducing agent 2MEA can improve discrimination accuracy between the positive specimen and the negative specimen in the measurement of soluble GPC3. In addition, when the negative specimen was pretreated with 2MEA, the matrix difference approached 100% and the matrix effect was reduced. Therefore, it has been confirmed that the pretreatment of the specimen with the reducing agent 2MEA is useful for the measurement of soluble GPC3.

Example 3: Pretreatment of specimen with reducing agent TCEP in measurement of soluble GPC3

As a pretreatment liquid, a phosphate buffer containing 6.25 mM to 100 mM tris (2-carboxyethyl) phosphine hydrochloride (TCEP) (10 mM phosphate buffer, 6.25 to 100 mM TCEP, pH 6.0) was used. As a control liquid, a 10 mM phosphate buffer (pH 6.0) was used. Each of the negative specimen, the positive specimen, and the buffer sample was mixed with the pretreatment liquid at a volume ratio of 1 : 1, and reacted at 37°C for 6.5 minutes (with pretreatment). Similarly, each of the negative specimen, the positive specimen, and the buffer sample was mixed with the control liquid, and reacted (without pretreatment).
For each of the specimens, soluble GPC3 was measured by the measurement method described in Reference Example 4. The count of each of the specimens is presented in Table 3. In addition, as in Example 1, a lowering rate (%), a count difference (%) between the positive and the negative, and a matrix difference (%) were calculated.
Table 3. Pretreatment of specimens with TCEP

| | | Without Preteatmert | With Pretreatment | | |
|---|---|---|---|---|---|
| | TCEP (mM) | 0 | 6.25 | 50 | 100 |
| Count | NS-3<br>NS-4 | 5,747<br>7,418 | 2,824<br>3,251 | 3,470<br>2,709 | 2,017<br>1,400 |
| | PS-1<br>PS-2 | 18,169<br>22,033 | 13,649<br>18,215 | 15,131<br>10,897 | 17,812<br>5,596 |
| Lowering rate (%) | NS-3<br>NS-4 | | 50.9<br>562 | 39.6<br>63.5 | 64.9<br>81.1 |
| | PS-1<br>PS-2 | | 24.9<br>17.3 | 16.7<br>50.5 | 2.0<br>74.6 |
| Count difference (%) between positive and negative | PS-1,2 / NS-3,4 | 305 | 525 | 421 | 685 |
| Count | BS<br>Mean of NS-3.4 | 1,336<br>6,583 | 1,486<br>3,038 | 1,768<br>3,090 | 2,216<br>1,709 |
| Matrix difference (%) | BS /Mean of NS-1.2 | 20 | 49 | 57 | 130 |
| NS: Negative Specimen<br>PS: Positive Specimen<br>BS: Buffer Sample | | | | | |

As presented in Table 3, when the negative specimen was pretreated with TCEP, the count of the negative specimen decreased at each of the examined concentrations (6.25 mM to 100 mM), and a lowering rate thereof was very large. Meanwhile, when the positive specimen was pretreated with TCEP, the count of the positive specimen did not decrease or a lowering rate thereof was smaller than that of the negative specimen even if the count decreased. This indicates that the pretreatment of the specimen with the reducing agent TCEP can improve discrimination accuracy between the positive specimen and the negative specimen in the measurement of soluble GPC3. In addition, when the negative specimen was pretreated with TCEP, the matrix difference approached 100% and the matrix effect was reduced. Therefore, it has been confirmed that the pretreatment of the specimen with the reducing agent TCEP is useful for the measurement of soluble GPC3.

(Summary of Examples 1 to 3)

In consideration of the results of Examples 1 to 3, it is considered that the pretreatment of a specimen with a reducing agent can improve discrimination accuracy between a positive specimen and a negative specimen in the measurement of soluble GPC3 regardless of the type and structure of the reducing agent. In addition, it is considered that the pretreatment of the negative specimen with the reducing agent can output a true value independent of the type of specimen by reducing the matrix effect. Therefore, the pretreatment of a specimen with the reducing agent is useful for the measurement of soluble GPC3.

Example 4: Pretreatment of specimen with combination of reducing agent and surfactant in measurement of soluble GPC3

As pretreatment liquids, phosphate buffers (pH 6.0) of Test Examples 2 to 11 presented in Table 4A were used. The phosphate buffers of Test Examples 2 to 11 each contained DEAET as a reducing agent and 3-[(3-cholamidopropyl) dimethylammonio]-1-propanesulfonate (CHAPS) or polyoxyethylene sorbitan monolaurate (Tween 20) as a surfactant. As a control liquid, a 10 mM phosphate buffer (pH 6.0) of Test Example 1 presented in Table 4A was used. Each of the negative specimen, the positive specimen, and the buffer sample was mixed with the pretreatment liquid at a volume ratio of 1 : 1, and reacted at 37°C for 6.5 minutes (with pretreatment). Similarly, each of the negative specimen, the positive specimen, and the buffer sample was mixed with the control liquid, and reacted (without pretreatment).
For each of the specimens, soluble GPC3 was measured by the measurement method described in Reference Example 4. The count of each of the specimens is presented in Table 4B. In addition, as in Example 1, a lowering rate (%), a count difference (%) between the positive and the negative, and a matrix difference (%) were calculated.

[Table 4]

Table 4. Pretreatment of specimens with combination of reducing agent with surfactant

| A) Composition of pretreatment solution | | | | | | | | | | | | | |
|---|---|---|---|---|---|---|---|---|---|---|---|---|---|
| Test Example | Concentration | 1 | 2 | 3 | 4 | 5 | 6 | 7 | 8 | 9 | 10 | 11 | 12 |
| Phosphate | Molarity (mM) | 10 | 10 | 10 | 10 | 10 | 10 | 10 | 10 | 10 | 10 | 10 | 10 |
| DEAET | Molarity (mM) | 0 | 200 | 200 | 200 | 200 | 200 | 200 | 200 | 200 | 200 | 200 | 200 |
| CHAPS | % by weight | 0 | 0 | 0.1 | 0.5 | 25 | 5 | 10 | 0 | 0 | 0 | 0 | 0 |
| Tween20 | % by weight | 0 | 0 | 0 | 0 | 0 | 0 | 0 | 0.1 | 0.5 | 25 | 5 | 10 |
| B) Experimental results | | | | | | | | | | | | | |
|  | Test Example | 1 | 2 | 3 | 4 | 5 | 6 | 7 | 8 | 9 | 10 | 11 | 12 |
| Count | NS-1<br>NS-2 | 11,875<br>13,625 | 5,329<br>4,562 | 4,170<br>3,504 | 3,795<br>3,266 | 2,384<br>2,425 | 2,447<br>2,383 | 2,323<br>2,140 | 4,244<br>3,367 | 3,789<br>3,159 | 3,152<br>2,893 | 2,617<br>2,593 | 2,476<br>2,495 |
|  | PS-1<br>PS-2 | 21,323<br>29,252 | 19,440<br>26,749 | 15,190<br>19,359 | 13,534<br>19,059 | 9,494<br>14,045 | 6,018<br>8,169 | 4,597<br>6,144 | 14,800<br>19,610 | 13,579<br>18,841 | 12,564<br>17,907 | 9,970<br>14,199 | 9,975<br>13,907 |
| Lowering rate (%) | NS-1<br>NS-2 | | 55.1<br>66.5 | 64.9<br>74.3 | 68.0<br>76.0 | 79.9<br>82.2 | 79.4<br>82.5 | 804<br>84.3 | 64.3<br>75.3 | 68.1<br>76.8 | 73.5<br>78.8 | 78.0<br>81.0 | 79.1<br>81.7 |
|  | PS-1<br>PS-2 | | 8.8<br>8.6 | 28.8<br>33.8 | 36.5<br>34.8 | 55.5<br>52.0 | 71.8<br>72.1 | 78.4<br>79.0 | 30.6<br>33.0 | 36.3<br>35.6 | 41.1<br>38.8 | 53.2<br>51.5 | 53.2<br>52.5 |
| Count difference (%) between positive and negative | PS-1,2 /NS-1,2 | 198 | 467 | 450 | 462 | 489 | 294 | 241 | 452 | 467 | 504 | 464 | 480 |
| Count | BS<br>Mean of NS-1.2 | 1,734<br>12.750 | 1,895<br>4.946 | 1,928<br>3.837 | 2,112<br>3.531 | 2,573<br>2.405 | 2,560<br>2.415 | 2,654<br>2232 | 1,965<br>3,806 | 2,039<br>3,474 | 2,035<br>3,023 | 2,372<br>2,605 | 2,328<br>2,486 |
| Matrix difference (%) | BS /Mean of NS-1,2 | 14 | 38 | 50 | 60 | 107 | 106 | 119 | 52 | 59 | 67 | 91 | 94 |
| NS: Negative Specimen<br>PS: Positive Specimen<br>BS: Buffer Sample | | | | | | | | | | | | | |

As presented in Table 4, when the negative specimen was pretreated with a combination of a reducing agent and a surfactant, the count of the negative specimen decreased at each of the examined surfactant concentrations (0.1 to 10% by weight), and a lowering rate thereof was very large. Meanwhile, when the positive specimen was pretreated with a combination of a reducing agent and a surfactant, a lowering rate of the count of the positive specimen was smaller than that of the negative specimen. In particular, when a surfactant was added at a predetermined concentration (0.1 to 2.5% by weight CHAPS or 0.1 to 10% by weight Tween 20), the count difference (%) between the positive and the negative was very large. In addition, when the negative specimen was pretreated with a combination of a reducing agent and a surfactant, the matrix difference approached 100% and the matrix effect was further reduced. This indicates that the pretreatment of the specimen with a combination of a reducing agent and a surfactant can improve discrimination accuracy between the positive specimen and the negative specimen in the measurement of soluble GPC3 and that the matrix effect can be reduced. Therefore, it has been confirmed that the pretreatment of the specimen with a combination of a reducing agent and a surfactant is useful for the measurement of soluble GPC3.

[SEQUENCE LISTING]

**Claims**

1.   A method for treating a soluble GPC3-containing specimen in soluble GPC3 immunoassay, the method comprising mixing the soluble GPC3-containing specimen with a reducing agent.

2.   The method according to claim 1, wherein the reducing agent is used at a final concentration of 0.05 to 1,000 mM.

3.   The method according to claim 1 or 2, comprising further mixing the soluble GPC3-containing specimen with a surfactant.

4.   The method according to claim 3, wherein the surfactant is used at a final concentration of 0.005 to 10% by weight.

5.   The method according to any one of claims 1 to 4, wherein the immunoassay is sandwich immunoassay using two or more different kinds of antibodies against two or more different kinds of epitopes in soluble GPC3.

6.   The method according to any one of claims 1 to 5, wherein the soluble GPC3-containing specimen is a blood specimen.

7.   The method according to any one of claims 1 to 6, wherein the soluble GPC3-containing specimen is obtained from a subject suffering from cancer.

8.   The method according to claim 7, wherein the cancer is liver cancer.

9.   A soluble GPC3 immunoassay method comprising the following (a) and (b):

   (a) mixing a soluble GPC3-containing specimen with a reducing agent; and
   (b) measuring the amount of soluble GPC3 in the mixture obtained in (a) using one or more kinds of antibodies against soluble GPC3.

10.   The method according to claim 9, comprising further mixing the soluble GPC3-containing specimen with a surfactant.

11.   The method according to claim 9 or 10, wherein the measurement is performed by sandwich immunoassay using two or more different kinds of antibodies against two or more different kinds of epitopes in soluble GPC3.

12.   A soluble GPC3 immunoassay reagent comprising the following (a) and (b):

   (a) a reducing agent; and
   (b) one or more kinds of antibodies against soluble GPC3.

13.   The immunoassay reagent according to claim 12, further comprising (c) a surfactant.

14.   The immunoassay reagent according to claim 12 or 13, wherein the one or more kinds of antibodies against soluble GPC3 are two or more different kinds of antibodies against two or more different kinds of epitopes in soluble GPC3, and the immunoassay reagent is a reagent for sandwich immunoassay.

15. The immunoassay reagent according to any one of claims 12 to 14, wherein the reagent is a reagent for diagnosing cancer.

# FIG. 1

← Full-length (N-terminal fragment + C-terminal fragment)

← N-terminal fragment

WB = Antibody A   WB = Antibody B

| No. | Antigen | Amount |
|-----|---------|--------|
| 1, 5 | HepG2 | 7.2 ng |
| 2, 6 | HepG2 | 1.4 ng |
| 3, 7 | rhGPC3 | 7.2 ng |
| 4, 8 | rhGPC3 | 1.4 ng |

## INTERNATIONAL SEARCH REPORT

| International application No. |
|---|
| **PCT/JP2022/001385** |

### A. CLASSIFICATION OF SUBJECT MATTER

*C07K 16/18*(2006.01)i; *G01N 33/531*(2006.01)i; *G01N 33/574*(2006.01)i; *C07K 14/47*(2006.01)i
FI: G01N33/531 B; C07K14/47; C07K16/18; G01N33/574 A ZNA

According to International Patent Classification (IPC) or to both national classification and IPC

### B. FIELDS SEARCHED

Minimum documentation searched (classification system followed by classification symbols)

C07K14/47; C07K16/18; G01N33/531; G01N33/574

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| A | WO 2004/038420 A1 (PERSEUS PROTEOMICS INC) 06 May 2004 (2004-05-06) claims 1, 5, 7 | 1-15 |
| A | WO 2015/097928 A1 (CHUGAI PHARMACEUTICAL CO LTD) 02 July 2015 (2015-07-02) paragraph [0001] | 1-15 |
| A | WO 2019/107279 A1 (FUJIREBIO INC) 06 June 2019 (2019-06-06) claim 1 | 1-15 |
| A | WO 2005/040815 A1 (ADVANCED LIFE SCIENCE INSTITUTE, INC) 06 May 2005 (2005-05-06) claim 2 | 1-15 |
| A | WO 2018/051965 A1 (FUJIREBIO INC) 22 March 2018 (2018-03-22) claims 1, 2 | 1-15 |
| A | MAST, Alan. E. et al. Glypican-3 is a binding protein on the HepG2 cell surface for tissue factor pathway inhibitor. Biochemical journal. 1997, vol. 327, no. 2, pp. 577-583 abstract | 1-15 |

☐ Further documents are listed in the continuation of Box C.    ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **03 March 2022** | **05 April 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

## INTERNATIONAL SEARCH REPORT

| International application No. |
| --- |
| **PCT/JP2022/001385** |

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

    a.  ☑  forming part of the international application as filed:

          ☑  in the form of an Annex C/ST.25 text file.

          ☐  on paper or in the form of an image file.

    b.  ☐  furnished together with the international application under PCT Rule 13*ter*.1(a) for the purposes of international search only in the form of an Annex C/ST.25 text file.

    c.  ☐  furnished subsequent to the international filing date for the purposes of international search only:

          ☐  in the form of an Annex C/ST.25 text file (Rule 13*ter*.1(a)).

          ☐  on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
Information on patent family members

International application No.

**PCT/JP2022/001385**

| Patent document cited in search report | | | Publication date (day/month/year) | Patent family member(s) | | | Publication date (day/month/year) |
|---|---|---|---|---|---|---|---|
| WO | 2004/038420 | A1 | 06 May 2004 | US 2006/0014223 | A1 | | |
| | | | | claims 1, 5, 7 | | | |
| | | | | US 2015/0132782 | A1 | | |
| | | | | WO 2004/023145 | A1 | | |
| | | | | EP 1548442 | A1 | | |
| | | | | CA 2497418 | A1 | | |
| | | | | CN 1678911 | A | | |
| | | | | KR 10-2005-0057205 | A | | |
| WO | 2015/097928 | A1 | 02 July 2015 | US 2017/0010270 | A1 | | |
| | | | | paragraph [0001] | | | |
| | | | | EP 3088890 | A1 | | |
| | | | | CN 105849562 | A | | |
| WO | 2019/107279 | A1 | 06 June 2019 | US 2020/0371104 | A1 | | |
| | | | | claim 1 | | | |
| | | | | EP 3719499 | A1 | | |
| | | | | CN 111417854 | A | | |
| WO | 2005/040815 | A1 | 06 May 2005 | US 2008/0044807 | A1 | | |
| | | | | claim 2 | | | |
| | | | | EP 1691198 | A1 | | |
| | | | | CA 2544185 | A1 | | |
| | | | | CN 1871518 | A | | |
| | | | | KR 10-2006-0061400 | A | | |
| WO | 2018/051965 | A1 | 22 March 2018 | EP 3514539 | A1 | | |
| | | | | claims 1, 2 | | | |
| | | | | CN 109564214 | A | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2004038420 A
- WO 2015097928 A
- WO 2004022739 A
- WO 2007081790 A
- WO 2005039380 A

**Non-patent literature cited in the description**

- Detection of glypican-3-specific CTLs in chronic hepatitis and liver cirrhosis. *Oncology Reports,* 2009, vol. 22, 149-54